**Europäisches Patentamt**

**European Patent Office**

**Office européen des brevets**

(19)

(11) Veröffentlichungsnummer : **0 190 446 B2**

(12)

# NEUE EUROPÄISCHE PATENTSCHRIFT

(45) Veröffentlichungstag der neuen Patentschrift :
29.01.92 Patentblatt 92/05

(51) Int. Cl.⁵ : **A61F 2/32**

(21) Anmeldenummer : **85115869.1**

(22) Anmeldetag : **12.12.85**

(54) **Metallenes Knochenimplantat.**

(30) Priorität : **07.02.85 CH 558/85**

(43) Veröffentlichungstag der Anmeldung :
**13.08.86 Patentblatt 86/33**

(45) Bekanntmachung des Hinweises auf die
Patenterteilung :
**22.03.89 Patentblatt 89/12**

(45) Bekanntmachung des Hinweises auf die
Entscheidung über den Einspruch :
**29.01.92 Patentblatt 92/05**

(84) Benannte Vertragsstaaten :
**AT DE FR GB IT**

(56) Entgegenhaltungen :
**EP-A- 0 065 481**
**EP-A- 0 066 092**
**EP-A- 0 075 378**
**DE-A- 2 933 237**
**FR-A- 1 287 526**

(73) Patentinhaber : **GEBRÜDER SULZER**
**AKTIENGESELLSCHAFT**
**Zürcherstrasse 9**
**CH-8401 Winterthur (CH)**

(72) Erfinder : **Frey, Otto**
**Wallrütistrasse 56**
**CH-8400 Winterthur (CH)**
Erfinder : **Semlitsch, Manfred, Dr.**
**Endlikerstrasse 86**
**CH-8400 Winterthur (CH)**

(74) Vertreter : **Sparing Röhl Henseler**
**Patentanwälte European Patent Attorneys**
**Rethelstrasse 123**
**W-4000 Düsseldorf 1 (DE)**

**EP 0 190 446 B2**

Jouve, 18, rue Saint-Denis, 75001 PARIS

## Beschreibung

Die Erfindung betrifft ein metallenes Knochenimplantat, vorzugsweise eine Gelenkendoprothese, das mindestens teilweise aus einem geschlossenen, die Aussenform des Implantates gestaltenden Hohlkörper besteht, der selbsttragend ausgelegt ist.

Um vor allem bei Gelenkendoprothesen an Gewicht zu sparen, ist es bekannt, derartige Prothesen als geschlossene Hohlräume auszubilden (FR-A-2 021313). Wie die massiven Prothesenteile werden die hohlen bisher aus Schmiede- und/oder Gussteilen hergestellt, wobei die geschlossenen Hohlkörper durch Zusammenschweissen einzelner Formteile gefertigt werden. Als Beispiel sei ein Gelenkkopf für eine Femurkopfprothese erwähnt, der aus einer Hohlkugel aus Gusswerkstoff und einer damit verschweissten Hülse aus einem Schmiedewerkstoff besteht (vgl. die unter Art. 54 (3) EPÜ fallende EP-A-0 172 262). Die Herstellung und Bearbeitung der bisherigen Prothesen, die mindestens teilweise einen Hohlkörper bilden, ist daher - besonders wenn es sich z.B. um Verankerungsschäfte mit einer beispielsweise aus Rippen bestehenden Struktur handelt - teuer und aufwendig.

Aus der DE-A-2 933 237 sind für Hüftgelenksprothesen selbsttragende Hohlschäfte bekannt, deren Wände aus Metall bestehen können, wobei zur gezielten Änderung der Festigkeiten des Schaftes von proximal nach distal kontinuierlich abnehmende Wandstärken vorgesehen sind; über die Herstellungsverfahren dieser bekannten Hohlschäfte werden keine Angaben gemacht.

Zur Annäherung der Materialeigenschaften, wie z.B. Festigkeit und Biegesteifigkeit, an diejenigen des kortikalen Knochens ist aus der EP-A-0 065 481 bekannt, Prothesenschäfte für Hüftgelenksprothesen als offene Hohlschäfte auszubilden, die aus dem Blech einer Titanlegierung oder einem Faser-Verbund-Werkstoff bestehen können, diese metallenen Hohlschäfte haben Wandstärken von 1 bis 3 mm. Ihre Wände sind darüberhinaus mit Durchbrüchen versehen. Schon allein die Fixierung des Prothesenkopfes und -halses auf diesen Schäften ist arbeitsaufwendig und teuer.

Aufgabe der Erfindung ist es, die Herstellung von Hohlkörper- Prothesen zu vereinfachen, ohne dabei eine unzulässige Einbusse an den geforderten mechanischen Eigenschaften in Kauf zu nehmen.

Diese Aufgabe wird mit der vorliegeden Erfindung dadurch gelöst, dass der Hohlkörper eine durch Drücken, Pressen oder Ziehen geformte Blechschale ist, deren Wandstärke entlang des formgestaltenden Umfanges mindestens annähernd konstant ist.

Die relativ dünne Blechschale, deren Wandstärke mit Vorteil 1 -3 mm beträgt - wobei die für die selbsttragende Eigenschaft notwendige Dicke des Bleches von der Form der Schale und dem gewählten Material abhängt, - kann auch bei komplizierten Aussenformen in einem Arbeitsgang so geformt werden, dass - abgesehen vom Polieren beispielsweise von Gelenkgleitflächen - keine Nachbearbeitung der Form mehr erforderlich ist. Wenn möglich werden dabei mit Vorteil zwei in einer Symmetrie- Ebene geteilte Halbschalen hergestellt, die in einem weiteren Arbeitsgang längs ihres Umfanges miteinander verschweisst werden.

Ein weiterer Vorteil des neuen Schalenkörpers ist sein gegenüber den bekannten Konstruktionen nochmals erheblich verringertes Gewicht.

Sollte es notwendig sein, die Widerstandsfähigkeit des Schalenkörpers vor allem gegenüber Biegebelastungen zu erhöhen, so ist es vorteilhaft, wenn die unter Umständen relativ kompliziert geformte Schale in ihrem Inneren mindestens einen Einsatz enthält, der eine einfache geometrische Gestalt haben kann. Weiterhin kann man den Hohlraum der Schale mit einem elastischen volumeninvarianten Material, z.B. einem Epoxy- Harz oder einem Silikonkautschuk, füllen.

Selbstverständlich ist es auch möglich, die Steifigkeit des Hohlkörpers durch andere Füllungen, z. B. durch leichte Materialien hoher Steifigkeit, wie Polyäthylen u.a., zu verbessern.

Eine bevorzugte Ausführungsform eines erfindungsgemässen Implantats ergibt sich, wenn die Schale aus superplastischem Material gefertigt ist.

Als Materialien für das neue Implantat eignen sich alle in der Implantat-Technik üblichen Metalle, aus denen Bleche der genannten Wandstärken mit ausreichender Festigkeit hergestellt werden können; geeignete superplastische Werkstoffe sind z.B. Alpha/Beta-Titanlegierungen.

Im folgenden wird die Erfindung anhand von Ausführungsbeispielen im Zusammenhang mit der Zeichnung näher erläutert.

Fig. 1 ist ein Längsschnitt durch eine Femurkopfprothese;

Fig. 2-4 sind Querschnitte durch die Prothese nach Fig. 1, in denen verschiedene Möglichkeiten für die Ausbildung des Schalenhohlraumes gezeigt sind;

Fig. 5 schliesslich ist ein Schnitt durch eine künstliche Hüftgelenkspfanne.

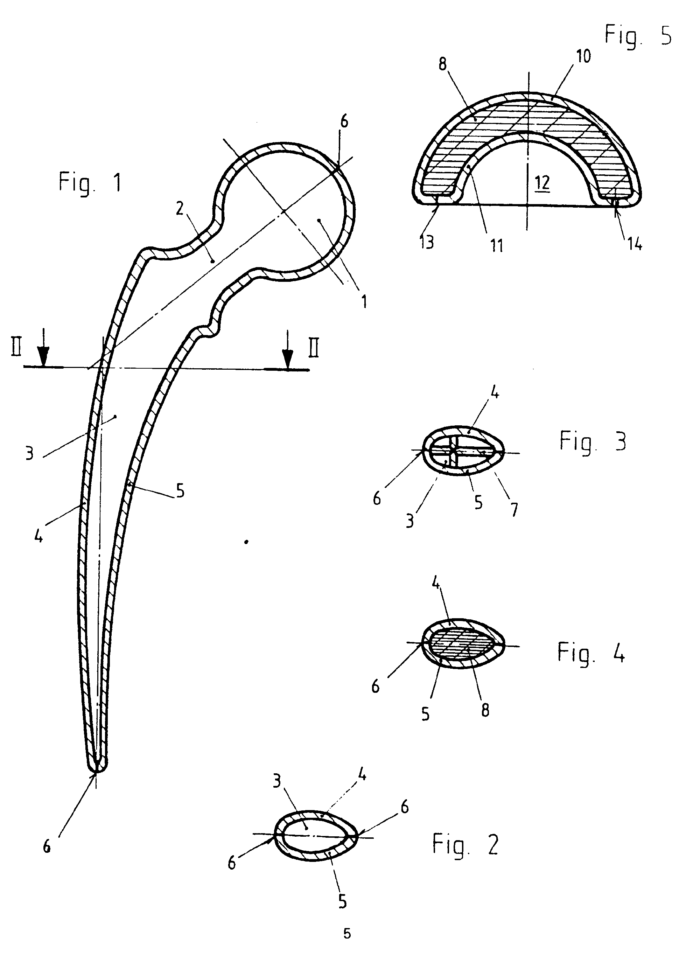

Wie der Schnitt nach Fig. 1 zeigt, ist die aus Gelenkkopf 1, Prothesenhals 2 und Verankerungsschaft 3 bestehende Femurkopfprothese aus zwei Halbschalen 4 und 5 zusammengesetzt; diese durch eine der bekannten Verformungsmethoden für Bleche gefertigten Halbschalen 4 und 5 haben gemäss der Erfindung

2

längs des ganzen Umfangs der Prothese mindestens annähernd die gleiche Wandstärke, die mit Vorteil zwischen 1 und 3 mm beträgt. Sie sind längs eines « Aequator »- Umfangs durch eine Schweissnaht 6 zu einem geschlossenen Hohlkörper verbunden. Als Werkstoffe für die Halbschalen eignen sich alle in der Implantat-Technik üblichen Metalle und Metall-Legierungen, die zu und als Bleche genügender Festigkeit zu verarbeiten sind. Der Hohlraum des geschlossenen Schalenkörpers 4, 5 kann je nach den Erfordernissen zur Erzielung einer erhöhten Festigkeit mit einem Einsatz 7 (Fig. 3) ganz oder teilweise ausgefüllt sein, der beispielsweise aus zu einem Kreuz verbundenen Stegen aus dem gleichen Material besteht.

Eine Möglichkeit, die Festigkeit, vor allem die Biegefestigkeit, der Blechschale 4, 5 zu verbessern, besteht darin, den Hohlraum des Schalenkörpers ebenfalls ganz oder teilweise mit einem volumeninvarianten elastischen oder plastischen Material 8 zu füllen (Fig. 4). Als derartige volumeninvariante Füllungen eignen sich vor allem zähflüssig oder gelartig bleibende aushärtende Kunststoffe, wie z.B. Epoxy- Harze oder Silikonkautschuk. Die Füllung des Hohlraumes erfolgt dabei mit Vorteil im Bereich der Schweissnaht 6 durch eine oder mehrere, nicht dargestellte Bohrungen hindurch, die anschliessend, beispielsweise mit Schweissmaterial, verschlossen werden.

Selbstverständlich ist es möglich, beide Arten der « Füllung » des Schalenhohlraumes miteinander anzuwenden, wobei gegebenenfalls in dem Einsatz 7 nicht dargestellte Durchtrittsöffnungen für das volumenkonstante Material vorgesehen sind.

Die rein schematisch dargestellte Hüftgelenkspfanne nach Fig. 5 setzt sich ebenfalls aus zwei Halbschalen 10 und 11 zusammen, von denen die eine 10 den Aussenmantel der Pfanne bildet, während die andere 11 den eigentlichen Pfannenhohlraum 12 für die Aufnahme eines nicht gezeigten Prothesengelenkkopfes umschliesst. Beide Halbschalen 10 und 11 sind längs einer Naht 13, die in der Mitte der Aequatorringfläche des Pfannenkörpers vorgesehen ist, - aber auch an einer anderen geeigneten Stelle des Pfannenumfangs liegen kann, - miteinander verschweisst. Der Innenhohlraum enthält wiederum eine Kunststoff-Füllung 8, die in diesem Falle aus einem leichten Material hoher Steifigkeit, beispielsweise aus Polyäthylen u.a. besteht und durch die Bohrung 14 eingepresst worden ist. Diese ist anschliessend durch einen Pfropfen aus Schweissmaterial verschlossen worden.

## Patentansprüche

1. Metallenes Knochenimplantat, vorzugsweise Geleßkendoprothese, das aus einem geschlossenen, die Außenform des Implantats gestaltenden Hohlkörper besteht, der selbsttragend ausgelegt ist, wobei der Hohlkörper eine durch Drücken, Pressen oder Ziehen geformte Blechschale (4, 5) ist, deren Wandstärke entlang des formgestaltenden Umfangs des Hohlkörpers mindestens annähernd konstant ist.

2. Knockenimplantat nach Anspruch 1, dadurch gekennzeichnet, dass die Wandstärke der Blechschale (4,5) 1-3 mm beträgt.

3. Knochenimplantat nach Anspruch 1 oder 2, dadurch gekennzeichnet, dass die Schale (4,5) in ihrem Inneren mindestens einen Einsatz (7) enthält.

4. Knochenimplantat nach einem der Ansprüche 1 bis 3, dadurch gekennzeichnet, dass die Schale (4,5) mit einem elastischen, volumeninvarianten Material (8) gefüllt ist.

5. Knochenimplantat nach einem der Ansprüche 1 bis 4, dadurch gekennzeichnet, dass die Schale (4,5) aus superplastischem Material gefertig ist.

## Claims

1. A metal bone implant, preferably a replacement joint, embodied by a self-supporting closed holow member defining the external shape of the implant, the hollow member being a sheet metal shell (4, 5) shaped by pressing or extruding or drawing, the wall thickness of the shell being at least substantially contant along the shape-defining periphery.

2. An implant according to claim 1, characterised in that the wall thickness of the shell (4, 5) is from 1 to 3 mm.

3. An implant according to claim 1 or 2, characterised in that the shell (4, 5) receives at leat one insert (7) in its interior.

4. An implant according to any of claims 1 - 3, characterised in that the shell (14, 5) is filled with a resilient invariable-volume material (8).

5. An implant according to any of claims 1 - 4, characterised in that the shell (4, 5) is made of a superplastic material.

**Revendications**

1. Implant osseux en métal, de préférence endoprothèse articulée, consistant en un corps creux fermé qui détermine la configuration externe de l'implant et est de réalisation autoportante, le corps creux étant une calotte en tôle (4, 5) mise en forme par emboutissage, pressage ou étirage, dont l'épaisseur de paroi est au moins approximativement constante le long du pourtour déterminant la configuration.

2. Implant osseux selon la revendication 1, caractérisé par le fait que l'épaisseur de paroi de la calotte en tôle (4, 5) mesure entre 1 et 3 mm.

3. Implant osseux selon la revendication 1 ou 2, caractérisé par le fait que la calotte (4, 5) renferme au moins une pièce intégrée (7) dans son espace interne.

4. Implant osseux selon l'une des revendications 1 à 3, caractérisé par le fait que la calotte (4, 5) est comblée par un matériau (8) élastique, à volume invariable.

5. Implant osseux selon l'une des revendications 1 à 4, caractérisé par le fait que la calotte (4, 5) est fabriquée en un matériau doué de plasticité supérieure.

Fig. 5

Fig. 1

Fig. 3

Fig. 4

Fig. 2